# EUROPEAN PATENT APPLICATION

(11) **EP 4 809 915 A1**
(43) Date of publication of application: **23.09.2026**
(21) Application number: 25164465.4
(22) Date of filing: 18.03.2025
(51) Int. Cl.: A61B 5/06, A61B 5/00, A46B 15/00

(54) **PERSONAL CARE SYSTEM**

(71) Applicant: Koninklijke Philips N.V., 5656 AE Eindhoven (NL)
(72) Inventor: DEVANAHALLI BALAKRISHNA, Naveen, Eindhoven (NL)
(74) Representative: Philips Intellectual Property & Standards

(57) **Abstract**

A personal care system comprising a personal care appliance with one or more functional components, a tracking subsystem configured to determine a current usage location of the personal care appliance, and a control subsystem. The control subsystem includes one or more processors configured to compare the current usage location to a predetermined guidance sequence and control a functionality of at least one functional component of the personal care appliance in dependence upon the comparison. Controlling the functionality comprises changing a power consumption of the at least one functional component of the personal care appliance. The system enables adaptive power management based on usage location adherence to a guidance sequence.

## Description

### FIELD OF THE INVENTION

The present invention relates to personal care appliances, and in particular, to personal care appliances having position tracking functionality.

### BACKGROUND OF THE INVENTION

In the context of personal care appliances, such as electric toothbrushes, effectively guiding users to follow recommended usage techniques remains challenging. One issue is that users often develop habitual brushing patterns that may not adequately clean all areas of the mouth. While some devices attempt to provide guidance through timers or zone indicators, users may still neglect certain regions or spend insufficient time in key areas. In addition, maintaining proper brushing technique throughout a session can be difficult, especially for children or individuals with limited dexterity.

Another problem is that personal care appliances typically operate at constant power levels regardless of how they are being used. This can lead to wasted energy when the device is not actively engaged in cleaning. It may also result in excessive wear on components or potential discomfort for users in sensitive areas.

It has been appreciated that a personal care system is needed that overcomes one or more of these problems.

### SUMMARY OF THE INVENTION

The invention is defined by the claims.

An aspect of invention is a personal care system. The personal care system includes a personal care appliance comprising one or more functional components, a tracking subsystem configured to determine a current usage location of the personal care appliance, and a control subsystem comprising one or more processors. The control subsystem is configured to compare the current usage location to a predetermined guidance sequence and control a functionality of at least one functional component of the one or more functional components of the personal care appliance in dependence upon the comparison, wherein controlling the functionality comprises changing a power consumption of the at least one functional component of the personal care appliance.

This system enables more efficient energy usage of a personal care appliance while at the same time providing an efficient mechanism for guiding users to follow an optimal usage technique. For example, at least one benefit of the invention is the ability to reduce power consumption when the current usage location does not match the guidance sequence. This feature not only conserves energy but also serves as a haptic or tactile indicator to a user of the device when they deviate from recommended usage patterns. Conversely, the system can maintain or increase power consumption when the user follows the guidance sequence, reinforcing proper usage habits.

The principles of the invention are applicable to a wide variety of personal care appliances. The personal care appliance may be an oral care appliance. The personal care appliance may, by way of example, comprise a toothbrush, an interdental cleaning device such as a powered flosser device, or a powered brushing mouthpiece device.

The personal care appliance may be a grooming device. The personal care appliance may, by way of example, comprise a powered shaver device, an epilator device, or an IPL device.

In some embodiments, the control subsystem may be configured to reduce or deactivate power consumption of the at least one functional component when the current usage location does not match a current location in the predetermined guidance sequence. This feature allows the system to conserve power when the appliance is not being used as intended, potentially extending battery life. This also provides a prompt or indicator to a user of the device that they have deviated from the guidance path.

The control subsystem may be configured to maintain or increase or activate power consumption of the at least one functional component when the current usage location matches a current location in the predetermined guidance sequence. By maintaining or increasing power when the appliance is used correctly, this feature reinforces proper usage habits and ensures optimal performance during intended use.

In some embodiments, the personal care appliance may be an electric toothbrush, and the at least one functional component may comprise a brush oscillation motor. Controlling the functionality of the at least one functional component may comprise changing the motor speed of the brush oscillation motor.

For example, when the user deviates from the predetermined usage guidance, the brush oscillation motor may be reduced in power or deactivated. For example, the brush oscillation speed or amplitude may be reduced or deactivated. This provides a haptic or tactical prompt to a user that they have deviated from the guidance sequence. In addition, this helps conserve power when the user is not applying the brush head in the correct brushing location.

In some embodiments, the predetermined guidance sequence may comprise a series of usage locations and corresponding usage durations. For example, the system is configured to track at any given time whether the user has been in a current usage location longer than is stipulated by the predetermined guidance sequence.

In some embodiments, the tracking subsystem may be further configured to monitor one or more further usage parameters of the personal care appliance during use. By way of non-limiting example, the one or more further usage parameters may comprise at least one of: a brush scrubbing parameter, a brush pressure parameter, and a handle grip parameter. The predetermined guidance sequence may comprise expected values for the one or more usage parameters, and the control subsystem may be configured to control the functionality of the at least one functional component further in dependence upon a comparison of current values of the one or more usage parameters with the expected values in the predetermined guidance sequence. This feature enables the system to provide more nuanced guidance and control, taking into account multiple aspects of proper appliance usage beyond just location.

In some embodiments, the control subsystem may be configured to determine a usage duration for the current usage location and control the functionality of the at least one functional component based on the usage duration.

In some embodiments, the control subsystem is further configured to adjust one or more operational parameters of the personal care appliance based on the current usage location. For example, the one or more operational parameters may comprise at least one of oscillation frequency and oscillation amplitude.

In some embodiments, the system may further comprise a user interface configured to provide feedback to a user based on the comparison between the current usage location and the predetermined guidance sequence. In some embodiments, the user interface may comprise at least one of a visual indicator, an auditory indicator, and a haptic indicator. In some embodiments, the user interface may take the form of a mobile computing device communicatively connected to the personal care appliance.

In some embodiments, the system may further comprise a mobile device configured to communicate with the personal care appliance. In some embodiments, the control subsystem may be further configured to transmit usage data to the mobile device, the usage data comprising at least one of the current usage location and a usage duration.

In some embodiments, the tracking subsystem may comprise an inertial sensor configured to detect a position and orientation of the personal care appliance.

In some embodiments, the predetermined guidance sequence may be a personalized guidance sequence generated based on calibration tracking data obtained by the tracking subsystem during a calibration usage session. Personalized guidance sequences can account for individual user characteristics and habits, potentially leading to more effective guidance.

For example, the control subsystem may be operable to execute a calibration procedure, comprising acquiring tracking data by means of the tracking subsystem during a usage session of the device by a user, the tracking data forming a calibration tracking data set; and generating a personalized guidance sequence for the user based on the calibration tracking data set.

In some embodiments, the control subsystem may be further configured to classify a current usage session as valid or invalid based on a comparison of a tracking data set acquired over the course of the current usage session and the predetermined guidance sequence. This feature allows the system to provide feedback on overall session quality, potentially encouraging users to complete full, proper usage sessions and discouraging incomplete or improper use.

In some embodiments, the control subsystem may be configured to enter a low-power standby mode when the current usage location does not match a current location in the predetermined guidance sequence. Entering a low-power standby mode when the appliance is not being used as intended can significantly extend battery life while still allowing for quick resumption of full operational use.

In some embodiments, the system may comprise a camera configured to capture images during usage. The control subsystem may be further configured to deactivate an image capture functionality of the camera responsive to detecting that a current usage location does not match a current location in the predetermined guidance sequence.

This feature can help conserve power and protect user privacy by ensuring that images are only captured when the appliance is being used as intended.

In some embodiments, the system includes an image handling module, wherein the image handling module is configured to selectively store or discard images captured by the camera during use in dependence upon a current usage location of the personal care appliance.

In some embodiments, the personal care appliance is an oral care appliance, the oral care appliance comprises a mouth-receivable portion configured for being received in the mouth during operation, wherein the camera is carried by the mouth-receivable portion, and wherein the image handling module is configured to selectively discard images captured by the camera while the mouth-receivable portion is detected to be located outside of the mouth. In other words, the image handling module does not store or process anything other than tooth, gums or oral care related information. This clearly has an advantage in terms of efficient utilisation of storage capacity. It also has an advantage with regards to privacy, since image data relating to the user's face and home environment is not stored.

In some embodiments, the personal care appliance is an electric toothbrush, wherein the electric toothbrush comprises a handle portion and a brush head attachment, the brush head attachment releasably attachable to the handle portion, wherein the control subsystem is further configured to deactivate the at least one functional component when the brush head of the electric toothbrush is detected to not be attached to the handle portion.

In some embodiments, the personal care appliance is an electric toothbrush. The electric toothbrush may comprise a handle portion. The system may further comprise a set of two or more brush head attachments of different brush head types, each of the brush head attachments releasably attachable to the handle portion. In some embodiments, the control subsystem may be operable to detect which of the set of brush head attachments is attached to the handle portion, and wherein the previously discussed control of the functionality of the at least one functional component is further based on which of the set of brush head attachments is detected as being attached to the handle portion.

In some embodiments, the system further comprises a microphone. The control subsystem may be configured to process sound data output by the microphone and to detect sound data associated with running water, and wherein the control subsystem is configured to deactivate the at least one functional component when running water is detected. This may be used to encourage a user to switch off the running tap while brushing their teeth, to thereby conserve water.

In some embodiments, the system further comprises a user interface, and wherein the control subsystem is configured to provide a manual override functionality using the user interface permitting a user to deactivate said control of functionality based on the comparison of the current usage location to the predetermined guidance sequence.

In some embodiments, the control subsystem is further operable in a tutorial mode, wherein the tutorial mode is configured to guide a user through a usage sequence defined by the predetermined guidance sequence using controlled activation and deactivation of the at least one functional component.

In some embodiments, the control subsystem is further configured to record an appliance use count indicative of a cumulative number of usage sessions performed by the user, and wherein, responsive to the use count exceeding a predetermined count threshold, the control subsystem is configured to deactivate the at least one functional component. For example, the personal care appliance may comprise one or more consumable components which are attached as attachments to the device, and wherein at least one consumable component has a recommended lifetime defined by a pre-defined number of usage sessions.

In some embodiments, the personal care appliance is an electric toothbrush, wherein the personal care appliance comprises an integrated display, wherein the control subsystem is configured to reduce a display brightness setting and/or deactivate the display responsive to the current usage location being a location inside the mouth.

Another aspect of the invention is a method of controlling a personal care appliance. The method comprises determining, by a tracking subsystem, a current usage location of a personal care appliance comprising one or more functional components, comparing the current usage location to a predetermined guidance sequence, and controlling a functionality of at least one functional component of the personal care appliance in dependence upon the comparison, wherein controlling the functionality comprises changing a power consumption of the at least one functional component of the personal care appliance.

Another aspect of the invention is a computer program comprising machine instructions configured, when executed on a processor, to cause the processor to carry out a method in accordance with any embodiment detailed in this disclosure or in accordance with any claim.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 illustrates a flowchart showing a method for controlling a personal care appliance, in accordance with one or more embodiments of the invention;
FIG. 2 illustrates a block diagram of a system for controlling a personal care appliance, in accordance with one or more embodiments of the invention;
FIG. 3 illustrates a block diagram illustrating steps performed in controlling a personal care system, in accordance with example embodiments of the invention;
FIG. 4 illustrates an example personal care appliance in the form of an electric toothbrush, comprising a set of functional components, in accordance with one or more embodiments of the invention;
FIG. 5 illustrates an operating workflow for controlling power consumption of a personal care appliance, in accordance with one or more embodiments of the invention;
FIG. 6 illustrates a further example personal care appliance in the form of an electric toothbrush, comprising a set of functional components which include a camera and a microphone, in accordance with one or more embodiments of the invention; and
FIG. 7 illustrates an example system in accordance with one or more embodiments of the invention in which the functionality of the control subsystem is implemented wholly or partially by a processing device of a mobile computing device and/or a cloud-based server.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Embodiments of the invention provide a personal care system configured to control the functionality of personal care appliances based on usage patterns. A tracking subsystem is used to determine a current usage location, and a control subsystem is used to compare this location to a predetermined guidance sequence. Based on this comparison, the system is configured to dynamically adjust the power consumption of functional components within the appliance. This approach enables more efficient energy usage and at the same time provides an efficient mechanism for guiding users to follow optimal usage techniques.

The principles of the present invention are applicable across various types of personal care appliances. While particularly well-suited for electric toothbrushes, where a brush oscillation motor may be controlled as a function of compliance with a guidance sequence, the principles are also appliable to other devices such as shavers, epilators, or interdental cleaning tools. For all personal care appliances, there is benefit in reducing power use when the user has deviated from proper usage guidance, where such use may represent wasted energy, and where changing the functionality of at least one functional component can act as a prompt to a user to return to proper usage guidance.

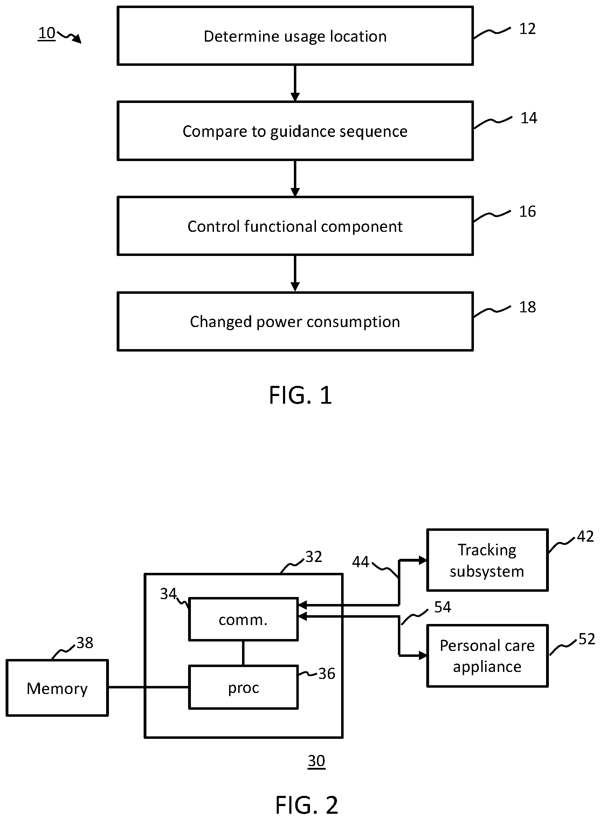

FIG. 1 illustrates an example method 10 for controlling a personal care appliance in accordance with one or more embodiments.

The method 10 may be implemented as a computer program comprising instructions executed on a processor.

The method comprises determining 12, by means of a tracking subsystem, a current usage location of a personal care appliance comprising one or more functional components. This step may involve using sensors or other tracking mechanisms to identify the position and orientation of the personal care appliance during use.

The method 10 further comprises comparing 14 the current usage location to a predetermined guidance sequence. The predetermined guidance sequence may, by way of example, include a series of expected usage locations and corresponding usage durations for use of the personal care appliance.

The method further comprises controlling 16 a functionality of at least one functional component of the personal care appliance. This control is performed in dependence upon the comparison between the current usage location and the predetermined guidance sequence. The step of controlling 16 the at least one function component is such as to result in changed power consumption 18 of the at least one functional component of the personal care appliance. By way of example, if the current usage location matches the expected location in the predetermined guidance sequence, the power consumption may be maintained or increased. Conversely, if the current usage location does not match the expected location, the power consumption may be reduced.

The method 10 may be repeated continuously or at regular intervals during the operation of the personal care appliance. This may for example help ensure ongoing adherence by the user to the predetermined guidance sequence and provides for more efficient power management since power consumption is reduced when the user is not correctly following the guidance sequence.

The step of controlling 16 the functionality of the at least one functional component so as to change its power consumption may encompass various approaches to modifying the energy usage of the personal care appliance. In some cases, this may involve reducing the power supplied to the functional component. For example, the control subsystem may decrease the voltage or current provided to a brush oscillation motor in an electric toothbrush, resulting in slower brush movements and lower power consumption.

In other instances, controlling 16 the functionality may comprise deactivating power to the functional component entirely. This could involve completely cutting off the electrical supply to a particular component, such as disabling the brush oscillation motor in an electric toothbrush or temporarily disabling a camera or other sensor when the appliance is not being used as intended. Deactivation may serve both to conserve energy and to encourage proper usage of the device.

The controlling 16 the functionality may additionally or alternatively comprise putting the personal care appliance into a low-power mode when the current usage location does not match the predetermined guidance sequence. In a low-power mode, non-essential functions may be disabled or operated at reduced capacity, while maintaining minimal functionality to allow for quick resumption of normal operation. For example, in an electric toothbrush, the brush oscillation motor may be slowed significantly, display brightness may be reduced and/or turned off, and wireless communication features (such as Wi-Fi or Bluetooth) may be temporarily disabled.

The specific implementation of the controlling 16 step may vary depending on the type of personal care appliance and its functional components. For instance, in a powered shaver, controlling the functionality might involve adjusting the speed of the cutting mechanism, while in an epilator, it could mean modifying the rotation speed of the tweezing cylinder.

As used herein, the term "usage location" may refer to a specific area or region on or within the body where a personal care appliance is performing a personal care function, for example where the personal care appliance is functionally engaging for a functional purpose, for example for a personal care function.

In the context of an electric toothbrush, a usage location might refer to one of a predetermined set of zones or quadrants within the oral cavity, such as the upper left, upper right, lower left, or lower right sections of the teeth and gums. A usage location might additionally or alternatively refer to one of a predetermined set of surfaces of the teeth, including the buccal (cheek-facing), lingual (tongue-facing), or occlusal (biting) surfaces. A usage location might additionally or alternatively refer to one of a predetermined set of regions of the mouth, such as the front teeth, molars, or along the gumline. A usage location might additionally or alternatively refer to one of a predetermined set of individual teeth or small groups of adjacent teeth being targeted for cleaning. A usage location might additionally or alternatively refer to one of a predetermined set of soft tissue areas within the mouth, for example including the tongue, inner cheeks, or roof of the mouth.

For a shaving device, a usage location might refer to one of a predetermined set of areas of the face, such as the cheeks, chin, upper lip, or jawline, one of a predetermined set of sections of the neck, including the front, sides, or under the chin, and/or one of a predetermined set of body regions where hair removal is desired, such as the legs, underarms, or chest.

As used herein, the term "guidance sequence" may refer to a predetermined series of usage locations, actions, and/or parameters that define an optimal or recommended usage pattern for a personal care appliance. The guidance sequence may be designed to ensure thorough and effective use of the personal care appliance, promoting proper technique and coverage of all relevant areas during a usage session.

In some embodiments, a guidance sequence may include a series of specific usage locations, each associated with a recommended usage duration. For example, in the context of an electric toothbrush, a guidance sequence may divide the mouth into multiple zones or quadrants, specifying the order in which these zones should be brushed and how long the user should spend on each zone. The sequence may also include recommendations for brushing different surfaces of the teeth, such as the outer, inner, and chewing surfaces.

In some cases, the guidance sequence may incorporate additional parameters beyond just location and duration. These may include recommended pressure levels, movement patterns, or angles of application for the personal care appliance. For instance, a guidance sequence for an electric shaver may specify different angles and directions of stroke for various facial areas to achieve the closest and most comfortable shave.

The guidance sequence may be customizable or adaptable based on individual user needs or preferences. As will be explained later, in some implementations, the system may allow for the creation of personalized guidance sequences based on data collected during a calibration session or over time through regular use of the appliance. This personalization may account for factors such as the user's oral anatomy, skin sensitivity, or specific grooming needs.

By way purely of non-limiting illustration, some examples of guidance sequences might be as follows.

For an electric toothbrush: A sequence that guides the user to brush the outer surfaces of the upper right teeth for 30 seconds, then the inner surfaces for 20 seconds, followed by the chewing surfaces for 10 seconds, before moving to the upper left quadrant and repeating the pattern, and then proceeding to the lower jaw.

For a facial cleansing brush: A sequence that directs the user to start with the forehead for 20 seconds, move to each cheek for 15 seconds each, then the nose and chin for 10 seconds each, finishing with gentle circular motions around the eyes for 5 seconds each.

For an electric shaver: A sequence that guides the user to shave the cheeks using downward strokes for 30 seconds, the chin and jawline using upward strokes for 20 seconds, the upper lip area for 10 seconds, and finally the neck area using upward strokes for 20 seconds.

In each of these examples, the guidance sequence provides a structured approach to using the personal care appliance, aiming to ensure thorough and effective coverage of all relevant areas while promoting proper technique and optimal results.

FIG. 2 illustrates a personal care system 30. The personal care system 30 includes a control subsystem 32, a tracking subsystem 42, and a personal care appliance 52.
The control subsystem 32 comprises one or more processors 36. The one or more processors 36 are configured to execute instructions for controlling the operation of the personal care system 30.

By way of one non-limiting example, the personal care appliance 52 may be an oral care appliance. The personal care appliance 52 may, by way of example, comprise a toothbrush, an interdental cleaning device such as a powered flosser device, or a powered brushing mouthpiece device. However, the personal care appliance 52 may be any other type of personal care appliance 52. By way of further non-limiting example, the personal care appliance 52 may comprise a powered shaver device, an epilator device, or an IPL device.

The personal care appliance 52 comprises one or more functional components.

The one or more functional components may include elements such as a brush oscillation motor or other components specific to the type of personal care appliance 52. The one or more functional components might for example comprise a shaving element driving motor for instance when the personal care appliance 52 is an electric shaver. The one or more functional components may include one or more sensors. As will be explained later, the one or more functional components might comprise a camera.

The tracking subsystem 42 is configured to determine a current usage location 44 of the personal care appliance 52. The tracking subsystem 42 may utilize various sensors or tracking mechanisms to identify the position and orientation of the personal care appliance 52 during use.

The control subsystem 32 receives information from the tracking subsystem 42 regarding the current usage location 44 of the personal care appliance 52. Based on this information, the control subsystem 32 can implement the method 10 described earlier, including the step of determining 12 a current usage location of the personal care appliance 52, the step of comparing 14 the current usage location to a predetermined guidance sequence, and the step of controlling 16 a functionality of at least one functional component of the one or more functional components of the personal care appliance 52 in dependence upon the comparison, which control results in the changed power consumption 18 of the personal care appliance 52.

FIG. 2 illustrates the control subsystem 32 of the personal care system 30. The control subsystem 32 includes a communication module 34, one or more processors 36, and a memory 38.

The communication module 34 facilitates data exchange between components of the personal care system 30. The communication module 34 may receive data from the tracking subsystem 42 regarding the current usage location 44 of the personal care appliance 52.

The one or more processors 36 execute instructions stored in the memory 38 to process information and generate control instructions 54. The control instructions 54 are sent to the personal care appliance 52 to control its functionality based on various factors.

As noted above, the control subsystem 32 compares the current usage location 44 to a predetermined guidance sequence stored in the memory 38. Based on this comparison, the control subsystem 32 controls the functionality of at least one functional component of the personal care appliance 52. This control involves changing the power consumption of the at least one functional component.

For example, in some embodiments, when the current usage location 44 matches an expected current location in the predetermined guidance sequence, the control subsystem 32 may maintain or increase power consumption of the at least one functional component. Conversely, when the current usage location 44 does not match an expected current location in the predetermined guidance sequence, the control subsystem 32 may reduce power consumption or enter a low-power standby mode.

In some embodiments, the predetermined guidance sequence may include a series of usage locations and corresponding usage durations. The control subsystem 32 may be configured to determine a usage duration for a current usage location 44 (or each current usage location) and control the functionality of the functional component based on this duration. A usage duration may mean a time duration that the personal care appliance 52 has been located or functionally engaged at the current usage location. For example if the current usage duration exceeds the expected usage duration for that usage location in the predetermined guidance sequence, the control subsystem 32 may be configured to control the functional component to reduce the power consumption, for example by reducing or deactivating the power to the functional component.

Additionally or alternatively, the control subsystem 32 may be configured to compare current values of one or more further usage parameters with expected values in the predetermined guidance sequence and to control the functionality of the functional component in dependence upon this comparison.

The tracking subsystem 42 is configured to determine the current usage location 44 of the personal care appliance 52. In some embodiments, the tracking subsystem 42 comprises an inertial sensor configured to detect a position and orientation of the personal care appliance 52. The inertial sensor may include components such as accelerometers and gyroscopes to measure linear acceleration and angular velocity, respectively. By processing data from these sensors, the tracking subsystem 42 can determine the spatial orientation and movement of the personal care appliance 52 during use. For example, inertial position tracking technology is well known in the field of oral care appliances, such as toothbrushes.

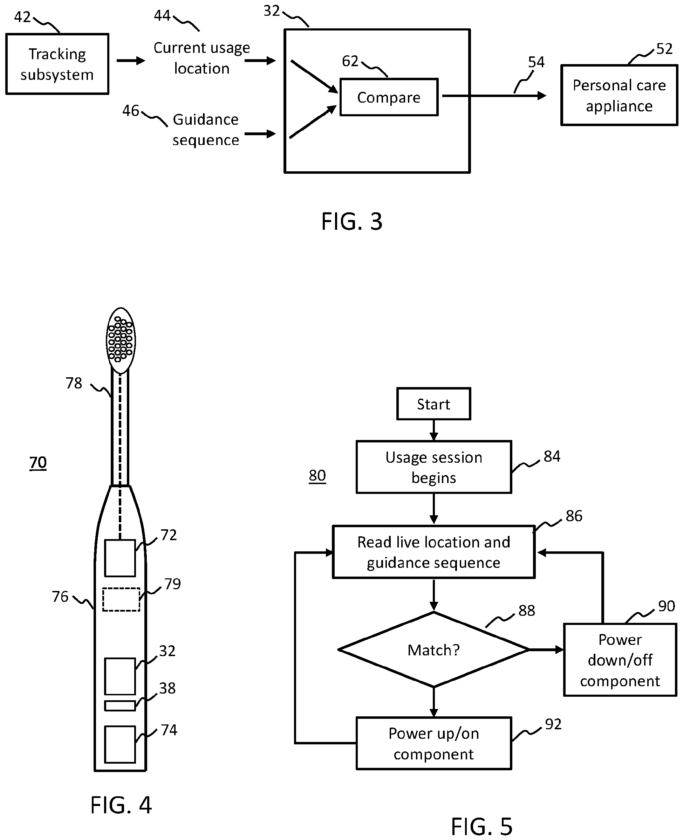

FIG. 3 illustrates an example processing flow, illustrating how the control subsystem 32 may process current location information 44 to control functionality.

The tracking subsystem 42 provides the current usage location 44 to the control subsystem 32. The control subsystem 32 uses this information in a comparison procedure 62 to compare the current usage location 44 with a guidance sequence 46. The comparison procedure 62 compares the current usage location 44 provided by the tracking subsystem 42 with a guidance sequence 46. The guidance sequence 46 may comprise a series of expected usage locations and corresponding usage durations. Using a timer, the control subsystem 32 may determine a total time duration from a start of the usage session, and may thereby determine an expected usage location at which the user should be using the device at a current time according to the guidance sequence 46.

Based on the comparison between the current usage location 44 and the guidance sequence 46, the control subsystem 32 generates control instructions 54. These control instructions 54 are sent to the personal care appliance 52 to adjust its functionality.

For example, if the current usage location 44 matches the expected location in the guidance sequence 46, the control instructions 54 may activate or maintain or increase power to the one or more functional components of the personal care appliance 52. Conversely, if the current usage location 44 deviates from the expected location, the control instructions 54 may reduce or deactivate power to the one or more functional components.

The control subsystem 32 continuously performs the comparison procedure 62 throughout the usage session, ensuring that the personal care appliance 52 operates in accordance with the guidance sequence 46. This dynamic adjustment allows for optimized performance and power management of the personal care appliance 52 while encouraging adherence to recommended usage patterns.

The guidance sequence 46 may comprise a series of usage locations and corresponding usage durations or timings. In some implementations, the guidance sequence 46 may be a personalised guidance sequence 46. It may be generated based on data collected during a calibration usage session. This data may be referred to as calibration tracking data. During the calibration usage session, the tracking subsystem 42 records information relating to a usage pattern of a user over the course of a single usage session. This recorded data is then processed to create a guidance sequence 46 adapted to the specific characteristics and preferences of the user. Personalized guidance sequences can account for individual user characteristics and habits, potentially leading to more effective guidance.

The calibration data may provide insights into the geometry or shape of the anatomy to which the personal care appliance 52 is intended to be applied. For example, for an oral care device, the calibration data may capture information about the shape and structure of a user's oral cavity, such as the configuration of their teeth. The control subsystem can utilize the calibration data acquired during the calibration usage session to compute a representation of a contour of the user's dental arch. This computed representation can then be used to generate a guidance sequence 46 that defines a series of cleaning locations within the oral cavity along with corresponding timing durations for each location. The cleaning locations may correspond to predetermined zones or areas of the oral cavity, such as sections of the dental arch.

To implement this calibration and guidance generation process, the control subsystem may employ various algorithms and data processing techniques. For example, the system may use machine learning models trained on large datasets of oral cavity shapes and optimal brushing patterns. Such a model may be applied to the individual user's calibration data to create a personalized guidance sequence 46.

Furthermore, the calibration process may take into account factors beyond just physical anatomy. For instance, it may consider the user's brushing speed, pressure applied, and typical brushing duration. This allows for further personalization of the guidance sequence 46.

In some embodiments, the guidance sequence 46 may be determined at least in part based on input by a professional practitioner, for example a health practitioner. For example, in the case of an oral care device, the guidance sequence 46 may be determined based at least in part on input by a dental health practitioner. By way of further example, in the case of a grooming device, the guidance sequence 46 might be determined at least in part based on input by a stylist.

In some embodiments, the guidance sequence 46 may be generated by a practitioner and sent or transmitted to the user of the system 30 for utilisation when operating the system. For example, a practitioner may generate the guidance sequence 46 and upload the guidance sequence 46 to a server, e.g. a cloud server. A mobile computing device associated with the user of the system may be operable to communicate with the server, and to download the guidance sequence 46 generated by the practitioner. The mobile device may be communicatively connected with the personal care appliance 52 and the personal care appliance 52 and may be configured to transfer the guidance sequence 46 from the mobile device to a memory 38 of the processing device 32 of the system 30.

In some embodiments, the control subsystem 32 may be configured to infer a current user of the device using tracking data from the tracking subsystem 42. For example, the control subsystem may be configured to detect a usage pattern of the device, identify from the usage pattern one of a set of stored personalised guidance sequence 46s to which the usage pattern corresponds, each associated with a different user, and thereby determine which of a set of users is currently using the device.

In addition to determining the current usage location 44, the control subsystem 32 may be configured to monitor one or more further usage parameters of the personal care appliance 52 during use.

By way of example, these usage parameters may include one or more of: a pressure application parameter, and a handle grip parameter. The pressure application parameter relates to a pressure with which the personal care appliance 52 is applied to the body. For example, for an oral care appliance such as an electric toothbrush, this may be a brushing pressure parameter indicative of a pressure with which a brush head is applied to oral surfaces. The pressure application parameter may be measured, for example, using a separate pressure sensor integrated into the personal care appliance 52. For example, a brush pressure parameter may be measured using a pressure sensor arranged to sense a pressure of application of a brush head to oral surfaces.

The handle grip parameter is a parameter indicative, at minimum, of whether a user is currently gripping or holding a handle portion of the device. In other words, at minimum, the handle grip parameter indicates whether the user is currently holding the personal care appliance 52. This in turn provides an indication of whether the user is currently using the personal care appliance 52. The handle grip parameter may for example be determined using a capacitive touch sensor or optical proximity sensor on the handle of the personal care appliance 52.

In the case of an oral care appliance, the one or more usage parameters may include a brush scrubbing parameter. The brush scrubbing parameter may be indicative of a pattern or profile of a scrubbing action of the user using the toothbrush. For example, it may be indicative of an amplitude or frequency of brush scrubbing. The brush scrubbing parameter may be derived, for example, using inertial sensor data from an inertial sensor comprised by the position tracking subsystem 42. This may be used to quantify the brushing motion.

The guidance sequence 46 may include expected values for one or more usage parameters. The expected values may provide a reference point for optimal usage of the personal care appliance 52. The control subsystem 32 may be configured to compare the expected values for the one or more usage parameters with the current measured values for the one or more usage parameters. The control subsystem 32 may be configured to adjust operation of the personal care appliance 52 based on the comparison. The control subsystem 32 may be configured to adjust operation of the at least one functional component based on the comparison.

In some embodiments, both the current usage location 44 and the additional usage parameters are provided to the control subsystem 32, and the control 16 of the at least one functional component is performed based on both. For example, the control instructions 54 are generated based on both.

By way of example, in some embodiments, the control subsystem 32 may be configured to reduce or turn off power to the at least one functional component of the personal care appliance 52 in the case that the handle grip parameter indicates that the user is not currently holding the device.

In some embodiments, the control subsystem 32 may be configured to reduce or switch off power to the at least one functional component when a current application pressure exceeds an expected application pressure defined in the guidance sequence by a threshold amount and/or if a current application pressure falls below an expected application pressure defined in the guidance sequence by a threshold amount. This may encourage a user to dynamically adjust their application pressure so that it matches the guidance sequence

For example, if the personal care appliance 52 is an electric toothbrush, the control subsystem 32 may be configured to reduce or turn off power to the brush oscillation motor if the user is applying excessive pressure or insufficient pressure. This provides a tactile indicator to the user to guide them in applying the correct pressure at each given moment throughout the usage session.

In some embodiments, the comparison procedure 62 performed by the control subsystem 32 may consider multiple factors simultaneously. The current usage location 44 may be evaluated alongside other parameters such as the brush pressure, scrubbing intensity, and motor intensity, comparing these to the reference values established during the calibration usage session. This multi-factor analysis allows for a more comprehensive assessment of brushing technique and more nuanced control of the electric toothbrush 70.

In some embodiments, the control subsystem 32 may be configured to detect or sense one or more further conditions related to usage or device setup, and to control functionality of the appliance 52, for example to control the at least one functional component, in dependence upon the detected further conditions. By way of example, the control subsystem 32 may be configured to deactivate one or more functional components when a brush head is not attached to the oral care appliance 52. This ensures that power is not wasted running the brushing motor when no cleaning can be performed. Additionally or alternatively, the control subsystem 32 may be configured to deactivate one or more functional components when a brush head is attached which is of a type which is not suitable for a current usage scenario. A usage scenario may be detected or inferred based on one or more real-time usage parameters acquired using one or more sensors integrated in the personal care appliance 52. For example, an inertial sensor unit can be used to detect an orientation of a toothbrush, and from this can be detected which part of the oral anatomy the brush head is being applied to. If, for example, the control subsystem 32 detects that the brush head is being applied to the tongue, the control subsystem 32 may be configured to infer that the current usage scenario is cleaning of the tongue. The control subsystem 32 may check whether a currently attached brush head is a tongue cleaning brush head type. If not, the control subsystem may be configured to deactivate the at least one functional component.

Additionally or alternatively, in some embodiments, the personal care appliance 52 is a toothbrush, and the control subsystem 32 may be operable to detect presence or absence of toothpaste on a brush head of the toothbrush, and wherein the control subsystem is configured to deactivate one or more functional components when toothpaste is not detected. For example, the control subsystem may be configured to deactivate the brush oscillation motor responsive to detecting that toothpaste has not been applied to the brush head. This effectively prevents the user from performing a cleaning session in the event that they have forgotten to apply toothpaste. Presence or absence of toothpaste could be detected in different ways. In the case that the toothbrush includes a camera integrated in the personal care appliance, for example in the brush head of an electric toothbrush, image data acquired by the camera may be analyzed to detect presence or absence of toothpaste, e.g. of foam associated with presence of toothpaste. Additionally or alternatively, in some examples, presence or absence of toothpaste may be detected using brushing pressure data from a pressure sensor, wherein the pressure data can be analyzed to detect a smoothness of lateral motion of the brush across the tooth surfaces (e.g. degree of absence of stick-slip or start-stop), and wherein smoothness above a threshold is taken to be indicative of presence of toothpaste, since toothpaste assists in lubricating bristle motion against teeth.

In some embodiments, the control subsystem 32 is configured to record an appliance use count in the memory 38. When this count exceeds a predetermined threshold, the control subsystem 32 may deactivate one or more functional components of the personal care appliance 52. For example, the appliance use count may be indicative of a cumulative number of usage sessions performed by the user, and wherein, responsive to the use count exceeding a predetermined count threshold, the control subsystem is configured to deactivate the at least one functional component. For example, the personal care appliance 52 may comprise one or more consumable components which are attached as attachments to the device, and wherein at least one consumable component has a recommended lifetime defined by a pre-defined number of usage sessions. For example, the consumable component could be a brush head for a toothbrush or a cutting head for a shaver. The control subsystem 32 may be configured to prevent further use of the device, by deactivating power to one or more functional components, such as a brush motor or shaving head motor, once the user count has exceeded the predetermined count threshold.

By way of illustration of the principles of the invention, an example implementation in which the personal care appliance 52 is an electric toothbrush will now be described, with reference to FIG. 4.

FIG. 4 illustrates a personal care appliance 52 in the form of an electric toothbrush 70. The electric toothbrush 70 comprises a handle portion 76 and a brush head attachment 78. The handle portion 76 houses several components.

A brush oscillation motor 72 is housed in the handle portion 76 and is configured to provide a source of oscillating motion for the brush head attachment 78 during operation. The control subsystem 32 may control the functionality of the brush oscillation motor 72 by configuring a motor speed of the brush oscillation motor 72.

The control subsystem 32 may also be housed within the handle portion 76. The control subsystem 32 manages the operation of the electric toothbrush 70, including controlling the functionality of the brush oscillation motor 72 based at least in part on a comparison between the current usage location 44 and the guidance sequence 46.

Although in the illustrated example of FIG. 4, the control subsystem 32 is shown as being physically located within the personal care appliance 52 itself, this is not essential. In general, the control subsystem 32 may be separate to the personal care appliance 52. The control subsystem 32 may be implemented by a processing device of another unit or device. For example, the control subsystem may be implemented by a mobile computing device. The mobile computing device may be communicatively connected to the personal care appliance 52. For example, the personal care appliance 52 may comprise a wireless communication interface permitting communication with the mobile computing device, for example permitting transmission of data from the position tracking subsystem 42 and permitting communication of control instructions 54 for controlling the at least one functional component. By way of further example, the control subsystem 32 may be implemented by a cloud-based server. The personal care appliance 52 may comprise a wireless communication interface permitting communication with the cloud server, for example via an internet connection. The wireless communication interface may be a Wi-Fi interface permitting connection to the internet, or may be a wireless interface permitting communication with a mobile computing device, wherein the mobile computing device has an internet connection.

The memory 38 may also be housed within the handle portion 76. The memory 38 may store data such as the guidance sequence 46, usage history, and operational parameters for the electric toothbrush 70.

Also housed in the handle portion 76 is a power source 74 configured to provide power to the components of the electric toothbrush 70. The power source 74 may be a rechargeable battery or other suitable power supply.

The electric toothbrush 70 may include an integrated display 79. The integrated display 79 may be located on the handle portion 76 and can provide visual feedback to the user regarding the operation of the electric toothbrush 70, such as brushing time, battery level, or adherence to the guidance sequence 46.

In some embodiments, the system 30 may include a set of two or more brush head attachments 78 of different types. These different types of brush head attachments 78 may be designed for specific purposes, such as general cleaning, sensitive gums, whitening and/or tongue cleaning. The brush head attachments 78 are releasably attachable to the handle portion 76, allowing users to interchange them as needed. The control subsystem 32 may be configured to detect which of the set of brush head attachments 78 is attached to the handle portion 76. Based on this detection, the control subsystem 32 may adjust the control of the functionality of the brush oscillation motor 72 or other components of the electric toothbrush 70 to optimize performance for the specific type of brush head attachment 78 in use.

For example, each brush head attachment 78 may have a unique identifier, such as an RFID tag, QR code, or specific electrical contacts. The handle portion 76 may include a corresponding sensor to detect and identify the attached brush head. The control subsystem 32 may store a set of operational parameters for each brush head type in the memory 38. These parameters might include motor speed ranges, oscillation patterns, or power settings for example. When a brush head is attached, the control subsystem 32 may be configured to identify it and load the appropriate operational parameters. For example, a sensitive gum brush head might use lower speeds and gentler oscillation patterns compared to a whitening brush head. In some embodiments, the system 30 may comprise functionality permitting users to create profiles with preferred settings for each brush head type, stored in the memory 38. The control subsystem 32 may then apply these personalized settings when a specific brush head is detected.

In some embodiments, each brush head 78 may have a detectable brush head ID. For example, this may be detectable by detecting an RFID tag carried by the brush head using an RFID sensor of the toothbrush. The control subsystem 32 may be configured to identify a current user of the toothbrush 70 among a set of different registered users, for example based on communicating with a software application installed on a mobile device of a current user, wherein the software app identifies the current user. Each user may be associated with one or more brush head IDs, for example in a user database. The control subsystem 32 may be configured to check whether an ID of a currently attached brush head is a brush head associated with the current user. If not, the control subsystem may be configured to deactivate power to the at least one functional component, for example to the brush oscillation motor 72. In this way, the system can ensure that each brush head is only used by the correct user.

FIG. 5 illustrates an operating workflow 80 for controlling a personal care appliance 52. After the usage session begins 84, the system is configured to read 86 from the tracking subsystem 42 live location data indicative of a real time usage location of the personal care appliance 52. This is compared continuously or at regular intervals against a guidance sequence. A decision step 88 determines whether there is a match between the current usage location 44 and the guidance sequence 46. The decision step 88 comprises performing the comparison 14, 62 between the current usage location and the expected usage location defined in the guidance sequence mentioned earlier.

If the current usage location does not match the expected usage location defined in the guidance sequence, the method comprises a step 90 of adjusting functionality of at least one functional component of the personal care appliance 52, where this adjustment has the effect of reducing or switching off power consumption by the functional component. This reduction in power consumption may involve deactivating or reducing the power supplied to the functional component, for example to a brush oscillation motor in the case of an electric toothbrush, or to a shaver head drive motor in the case of an electric shaver.

On the contrary, if a match is detected at the decision step 88, the operating workflow 80 proceeds to a step 92 in which power consumption is maintained or increased or switched on to the functional component. This maintenance or increase or activation in power ensures that the personal care appliance 52 operates at optimal performance when used in accordance with the guidance sequence 46.

After either step 90 or step 92, the operating workflow 80 returns to the read 86 step to continue monitoring the current usage location 44 and guidance sequence 46. The operating workflow 80 continues a continuous cycle or loop of reading, comparing, and adjusting power throughout the usage session, implementing the step of controlling 16 the functional component to result in changed power consumption 18 as described in the method 10 of FIG. 1.

This continuous monitoring and adjustment of power consumption based on adherence to the guidance sequence 46 allows for efficient use of the personal care appliance 52 while encouraging proper usage techniques.

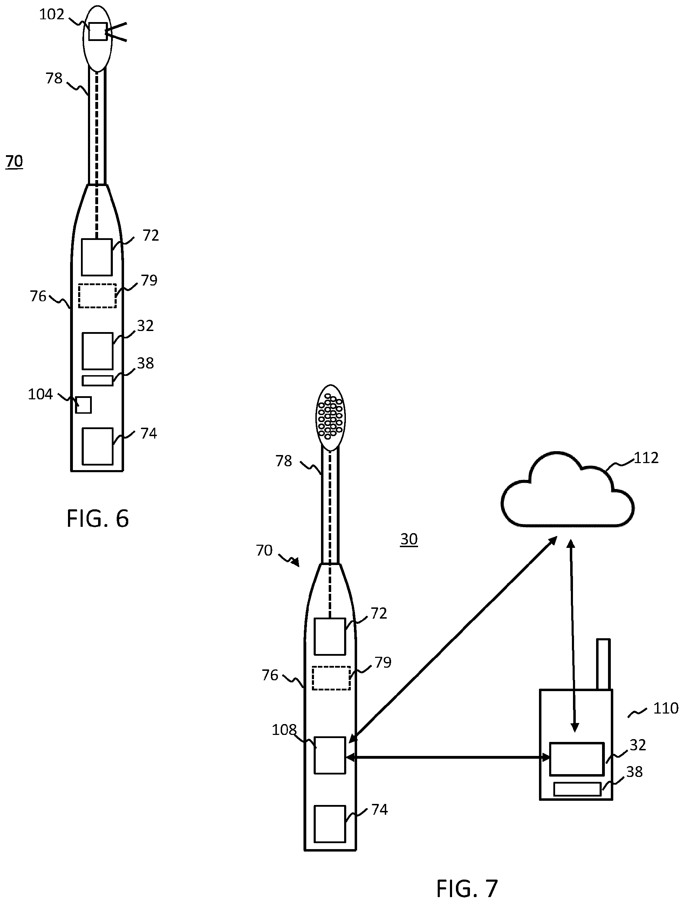

FIG. 6 illustrates a further example implementation of an embodiment of the invention, also comprising a personal care appliance 52 in the form of an electric toothbrush 70. **In** this example, the electric toothbrush is a variant of the toothbrush 70 described with reference to FIG. 4, comprising additional components and functionality.

Further to the components comprised by the toothbrush 70 of FIG. 4, the toothbrush 70 of FIG. 6 additionally comprises, a camera 102 positioned to capture images during usage. The camera 102 is arranged to capture images inside the oral cavity of the user during use. For example, the camera 102 is arranged to capture images of the user's teeth and gums during brushing sessions. The toothbrush 70 of FIG. 6 further comprises a sound sensor 104 configured to detect and process audio signals during the operation of the electric toothbrush 70. By way of example, the sound sensor 104 may be integrated in the handle portion 76 of the device. However, this is not essential.

The toothbrush 70 also comprises the brush oscillation motor 72, the control subsystem 32, the memory 38, the power source 74, and the display 79 which may be the same or similar to the descriptions provided with reference to FIG. 4.

In some embodiments, the control subsystem 32 may be configured to deactivate the image capture functionality of the camera 102 when the control subsystem 32 detects that the current usage location 44 does not match an expected current location in the guidance sequence 46. Deactivating the image capture functionality includes deactivating both static and video image capture functionality. This feature helps conserve power and storage resources when the electric toothbrush 70 is not being used in accordance with the recommended brushing pattern. In some embodiments, the control subsystem 32 may be configured to detect when the toothbrush head 78 is not located in the mouth of the user and to deactivate power to the camera 102 when the toothbrush head 78 is not located in the mouth of the user.

The system 30 may include an image handling module. The image handling module may be implemented as part of the control subsystem 32 or as a separate component within the electric toothbrush 70. The image handling module may be configured to selectively store or discard images and/or videos captured by the camera 102 based on the current usage location 44 of the electric toothbrush 70. For example, control subsystem 32 may be configured to detect when a current usage location does not match an expected usage location at a given time, communicate with the image handling module to communicate the non-match. The control subsystem 32 may be configured to detect when a current usage location does match an expected usage location at a given time and communicate with the image handling module to communicate the match. The image handling module may be configured to discard images or videos captured by the camera during a period where there is a non-match.

In some embodiments, the control subsystem 32 may be configured to detect when at least a mouth-receivable portion of the brush head is received within the mouth of the user during operation, and to detect when at least a mouth-receivable portion of the brush head is not received within the mouth of the user during operation and to communicate this to the image handling module. The image handling module may be configured to selectively discard images captured by the camera while the mouth-receivable portion is detected to be located outside of the mouth. In other words, the image handling module does not store or process anything other than tooth, gums, or oral care related information. This has an advantage in terms of efficient utilization of storage capacity. It also has an advantage with regards to privacy, since image data relating to the user's face and home environment is not stored.

The sound sensor 104 may function as a microphone, allowing the system 30 to capture and analyze audio data during brushing sessions. The control subsystem 32 may use the audio data from the sound sensor 104 to detect specific sounds associated with proper brushing technique or to identify potential issues such as excessive pressure or improper angle of brush head placement.

In some embodiments, the control subsystem 32 may be configured to process sound data output by the sound sensor 104 and to detect sound data associated with running water. The control subsystem 32 may be configured to deactivate the at least one functional component when running water is detected. For example, the control subsystem 32 may be configured to deactivate the brushing motor 72 when running water is detected. This may be used to encourage a user to switch off the running tap while brushing their teeth, to thereby conserve water.

By incorporating the camera 102 and the sound sensor 104, the electric toothbrush 70 provides additional data points for the control subsystem 32 to use in monitoring and guiding the user's operation of the personal care appliance 52.

In some embodiments, an integrated display on the handle portion 76 may be configured to adjust its brightness or power state based on the current usage location 44. For instance, when the tracking subsystem 42 determines that the brush head attachment 78 is inside the user's mouth, the control subsystem 32 may reduce the display brightness or turn off the display entirely to conserve power.

In some embodiments, the system 30 may be configured to incorporate user-specific risk factors into its operation. For instance, if a user profile stored in the memory 38 indicates that the user has recently undergone oral surgery or is a senior citizen, the control subsystem 32 may automatically adjust the intensity of the brush oscillation motor 72 or limit the use of high-intensity or deep clean modes to prevent potential harm.

In some embodiments, the system 30 may be configured to identify a current user of the personal care appliance 52 in the form of an electric toothbrush based on the characteristics of their oral cavity contour and brushing behaviors, as inferred using position data from the tracking subsystem 42. This feature could be particularly useful in households where multiple users share the same handle portion 76 but use different brush head attachments 78. The control subsystem 32 could automatically load the appropriate user profile and personalized guidance sequence 46 based on this identification, ensuring a tailored brushing experience for each user.

In some embodiments, the control subsystem 32 may be configured to classify each usage session as valid or invalid based on a comparison with the predetermined guidance sequence. This classification may be used for tracking and analysis purposes. For example, during each usage session, the tracking subsystem 42 may collect data relating to a user's brushing patterns, including locations brushed, duration spent at each location, and/or other relevant parameters such as pressure applied and/or brush movements. The control subsystem 32 may further employ a pre-defined algorithm to compare the collected usage data against the predetermined guidance sequence. This algorithm may consider factors such as coverage of all recommended usage locations (e.g. brushing zones), time spent in each zone compared to recommended durations, adherence to suggested brushing techniques or movements, and/or consistency of pressure applied. Based on the comparison, the control subsystem 32 may be configured to assign a score or percentage to indicate how closely the user followed the guidance sequence. The control subsystem 32 may be configured to apply one or more predefined thresholds for classifying a session as valid or invalid. For example, a session might be considered valid if it meets 80% or more of the guidance criteria. After classifying a session, the system may provide feedback to the user through various means such as the toothbrush display, a connected mobile app, or other user interface elements. The control subsystem 32 may store classification results over time, allowing for trend analysis and long-term tracking of user compliance with the guidance sequence.

In some embodiments, there may be provided a software application installed on a mobile computing device of a user, the software application including functionality to present to a user information about past usage sessions. For example, the software application may provide a graphical user interface presenting: information about progress over time in improving compliance with the guidance sequence, a calendar representation of usage session classifications over time, and/or representation of streaks of days in which the usage session was classified as valid.

In some embodiments, the control subsystem 32 may be operable in a tutorial mode, in which a user is guided through a usage sequence defined by the predetermined guidance sequence. This guidance is achieved through controlled activation and deactivation of functional components. The tutorial mode provides an interactive learning experience for users to become familiar with the proper usage technique for the personal care appliance 52. When activated, this mode systematically guides the user through each step of the predetermined guidance sequence, using the functional components of the appliance to provide real-time feedback and instruction.

For example, in an electric toothbrush implementation, the tutorial mode might work as follows. The control subsystem 32 activates the brush oscillation motor 72 when the tracking subsystem 42 detects that the toothbrush is in the correct starting position (e.g., upper right quadrant of the mouth). The motor remains active for the recommended duration for that quadrant, then deactivates to signal the user to move to the next position. The control subsystem 32 may use other functional components, such as LED indicators or haptic feedback, to guide the user to the next correct position. Once the tracking subsystem 42 detects the toothbrush in the correct new position, the brush oscillation motor 72 reactivates. This process continues, systematically guiding the user through all positions and durations specified in the predetermined guidance sequence.

In some embodiments, the system 30 may comprise a user interface allowing manual override of the functionality control based on the comparison of the current usage location 44 to the predetermined guidance sequence. This provides flexibility for users who may need to deviate from the standard guidance sequence. This might be implemented, for example, by providing a manual override option within a mobile application installed on a mobile computing device of the user.

As mentioned above, in some embodiments, the functionality of the control subsystem 32 may be partly or wholly implemented using a processing device comprised by a mobile computing device. FIG. 7 schematically illustrates an example implementation in accordance with such an embodiment. In the example shown in FIG. 7, the personal care appliance 52 is in the form of an electric toothbrush. The personal care appliance 52 comprises a local control module 108 comprising a wireless communication interface. The local control module is operatively coupled with the one or more functional components of the personal care appliance 52, such as the brush oscillation motor 72 and the display 79, and operable to control the one or more functional components to change a power consumption of each of the one or more functional components. In the illustrated example, the system further comprises a mobile computing device 110, wherein the mobile computing device comprises the control subsystem 32 and the memory 38 referred to previously with reference to FIG. 2, The control subsystem 32 is communicatively coupled with the wireless communication interface comprised by the local control module 108 of the personal care appliance 52 70. For example, with reference to FIG. 2, the control subsystem 32 may comprise a communication module 34, and wherein the communication module of the control subsystem 32 is arranged communicatively coupled with the wireless communication interface of the local control module 108 of the personal care appliance 52 for controlling the functionality of the one or more functional components of the personal care appliance 52.

As illustrated in FIG. 7, optionally the personal care appliance 52 and/or a mobile computing device 110 of the system 30 may be arranged communicatively linked with a cloud-based server 112. For example, in some embodiments, some or all of the functionality of the control subsystem 32 may be implemented by the cloud-based server. In some embodiments, the cloud-based server may be used for a data storage function, for example for storing the predetermined guidance sequence. The control subsystem 32 may be arranged communicatively coupled with the cloud-based server storage. Additionally or alternatively, the personal care appliance 52 may be arranged communicatively coupled with the cloud-based server 112. For example, if the control subsystem 32 is integrated in a mobile computing device 110, the control subsystem 32 and/or the wireless communication interface of the personal care appliance may be communicatively linked to the cloud-based server 112, for example for data retrieval purposes.

Embodiments of the invention described above comprise a control subsystem 32 employing one or more processors. It may be located in a single containing device, structure or unit, or it may be distributed between a plurality of different devices, structures or units. Reference therefore to the control subsystem being adapted or configured to perform a particular step or task may correspond to that step or task being performed by any one or more of a plurality of processing components, either alone or in combination. The skilled person will understand how such a distributed processing device can be implemented. The control subsystem includes a communication module or input/output for receiving data and outputting data to further components.

The one or more processors of the control subsystem can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A personal care system (30), comprising:
a personal care appliance (52) comprising one or more functional components;
a tracking subsystem (42) configured to determine (12) a current usage location (44) of the personal care appliance (52); and
a control subsystem (32) comprising one or more processors (36), the control subsystem (32) configured to:
compare (14) the current usage location (44) to a predetermined guidance sequence (46); and
control (16) a functionality of at least one functional component of the one or more functional components of the personal care appliance in dependence upon the comparison, wherein controlling the functionality comprises changing a power consumption of the at least one functional component of the personal care appliance.

2. The system of claim 1, wherein the control subsystem is configured to reduce power consumption of the at least one functional component when the current usage location (44) does not match a current location in the predetermined guidance sequence (46).

3. The system of claim 1 or 2, wherein the control subsystem is configured to maintain or increase power consumption of the at least one functional component when the current usage location (44) matches a current location in the predetermined guidance sequence (46).

4. The system of any one of claims 1 to 3, wherein the personal care appliance (52) is an electric toothbrush (70), and wherein the at least one functional component comprises a brush oscillation motor (72).

5. The system of claim 4, wherein controlling the functionality of the at least one functional component comprises changing a motor speed of the brush oscillation motor (72).

6. The system of any one of claims 1 to 5, wherein the predetermined guidance sequence (46) comprises a series of usage locations and corresponding usage durations.

7. The system of any one of claims 1 to 6,
wherein the tracking subsystem (42) is further configured to monitor one or more further usage parameters of the personal care appliance (52) during use,
wherein the predetermined guidance sequence (46) comprises expected values for the one or more usage parameters, and wherein the control subsystem (32) is configured to control the functionality of the at least one functional component further in dependence upon a comparison of current values of the one or more usage parameters with the expected values in the predetermined guidance sequence; and
optionally wherein the one or more further usage parameters comprise at least one of: a brush scrubbing parameter, a brush pressure parameter, and a handle grip parameter.

8. The system of any one of claims 1 to 7, wherein the control subsystem (32) is further configured to: determine a usage duration for the current usage location; and control the functionality of the at least one functional component based on the usage duration.

9. The system of any preceding claim, wherein the predetermined guidance sequence (46) is a personalized guidance sequence generated based on calibration tracking data obtained by the tracking subsystem during a calibration usage session.

10. The system of any preceding claim, wherein the control subsystem (32) is configured to enter a low-power standby mode when the current usage location (44) does not match a current location in the predetermined guidance sequence (46).

11. The system of any preceding claim, wherein the system further comprises a camera (102) configured to capture images during usage, wherein the control subsystem (32) is further configured to deactivate an image capture functionality of the camera responsive to detecting that a current usage location (44) does not match a current location in the predetermined guidance sequence (46).

12. The system of any preceding claim, further comprising a microphone (104), wherein the control subsystem (32) is configured to process sound data output by the microphone and to detect sound data associated with running water, and wherein the control subsystem (32) is configured to deactivate the at least one functional component when running water is detected.

13. The system of any preceding claim, wherein the control subsystem (32) is further operable in a tutorial mode, wherein the tutorial mode is configured to guide a user through a usage sequence defined by the predetermined guidance sequence (46) using controlled activation and deactivation of the at least one functional component.

14. A method (10) of controlling a personal care appliance (52), the method comprising:
determining (12), by a tracking subsystem (42), a current usage location of a personal care appliance comprising one or more functional components;
comparing (14) the current usage location to a predetermined guidance sequence; and
controlling (16) a functionality of at least one functional component of the personal care appliance in dependence upon the comparison, wherein controlling the functionality comprises changing a power consumption (18) of the at least one functional component of the personal care appliance.

15. A computer program comprising instructions which, when executed on a processor, cause the processor to carry out the method of claim 14.
